Europäisches Patentamt

European Patent Office

Office européen des brevets

(19)

(11) Veröffentlichungsnummer: **0 106 944**
A2

(12) **EUROPÄISCHE PATENTANMELDUNG**

(21) Anmeldenummer: 83107234.3

(22) Anmeldetag: 23.07.83

(51) Int. Cl.³: **B 01 J 8/28**
// C07C11/06, C07C5/333

(30) Priorität: 02.08.82 US 404284

(43) Veröffentlichungstag der Anmeldung: 02.05.84
Patentblatt 84/18

(84) Benannte Vertragsstaaten: BE DE FR GB IT LU NL

(71) Anmelder: **Institute of Gas Technology, 3424 South State Street, Chicago, Illinois 60616 (US)**

(72) Erfinder: **Kono, Hisashi O., 1616 Hinman Avenue Evanston, Illin. 60201 (US)**

(74) Vertreter: **Vogel, Georg, Hermann-Essig-Strasse 35 Postfach 105, D-7141 Schwieberdingen (DE)**

(54) Verfahren und Vorrichtung für ein fluidisiertes Feststoffsystem.

(57) Ein Verfahren und eine Vorrichtung zur Durchführung von Reaktionen in Fließbett-Feststoffsystemen, die in chemischen und thermischen Prozessen eingesetzt werden können, um homogene, dichte Fließbettbedingungen bei hohen Oberflächengasgeschwindigkeiten und reduzierter Rückmischung der Gase zu erhalten, verwenden eine Vielzahl vertikal angeordneter Fließbettzonen. Diese Fließbettzonen sind untereinander durch Fallrohre verbunden, die sich von dem unteren Teil einer höherliegenden Fließbettzone zu einer tieferliegenden Fließbettzone erstrecken, um eine hohe Feststoffzirkulation zu erreichen.

Verfahren und Vorrichtung für ein fluidisiertes Feststoffsystem

Die Erfindung betrifft ein Verfahren und eine Vorrichtung zur Durchführung von Reaktionen in fluidisierten Feststoffsystemen.

Fluidisierte Fließbetten von Feststoffen werden in einer großen Verschiedenheit von Prozessen für den verbesserten Flüssig-Fest-Kontakt eingesetzt, um physikalische Reaktionen, wie Wärmeübertragung, und chemische Reaktionen zwischen Gasen und Feststoffen einzuleiten und um ein Medium zum Leiten von chemischen Reaktionen unter der Verwendung von Feststoff-Katalysatoren zu haben.

Eine Hauptschwierigkeit bei bekannten Fließbettreaktoren liegt darin, aus Laboreinheiten eine industrielle Anlage aufzubauen. Die wichtigste Anforderung für einen erfolgreichen Aufbau liegt darin, ein homogenes Fließbett aufrecht zu erhalten, da ein nicht homogenes Fließbett die Größe des Fließbettes, das Verhalten der Feststoffe beeinflußt, wie von J.J. Van Heerden, in "Mixing Patterns in Large-Scale-Fluidized Bed" in J.R. Grace und J.F. Matsen Eds. "Fluidization", Seite 69, New York, Plenum Press, 1980 beschrieben ist. Ein homogenes Fließbett wird als solches definiert, in dem die Blasenbildung verhältnismäßig klein ist, der Zirkulationsstrom der Feststoffteilchen und der Gas-Feststoff-Kontakt intensiv sind und in dem die kleinste Blasenbildungsgeschwindigkeit größer ist als die kleinste Fluidisierungsgeschwindigkeit. Für die weitere Erläuterung der Arten der Gasfluidisierung siehe D. Geldart "Type of Gas Fluidization", Powder Technology 1, Seite 285, 1972. Feststoffteilchen mit einem mittleren Durchmesser von weniger als 100µ müssen verwendet werden, um einen homogenen Fluidisierungszustand zu erhalten, wie W.P.M. van Swaaij' in "Gas-Solids Fluid Bed Reactors", A.C.S. Symp. Ser. 72, Seite 1973 (1978) ausführt. Es hat sich jedoch gezeigt, daß selbst bei Verwendung von so kleinen Teilchen die unerwünschte Blasenbildung in dem Fließbett nicht verhindert werden kann, wie J. Yernshalmi und A.M. Squires in "The Phenomenon of Fast Fluidization", A.I.CH.E. Symp. Ser. 73, Seite 44 (1977) zeigen. In einem Versuch zur Lösung dieser Schwierigkeit verwenden Yernshalmi und Squires ein sehr schnelles Fließbett, das jedoch den Nachteil hat, daß keine dichte Phase auftritt und daß ein großer Anteil der Feststoffteilchen aus dem Fließbett getragen und über einen Abscheider, z. B. ein Zyklon, wieder zurückgeführt werden müssen. Die Anwendung von inneren Prallblechen zur Steuerung des Gasstromes in Fließbettreaktoren wurde von D. Harrison und J.R. Grace in J.F. Davidson und D. Harrison's "Fluidization", Seite 559, New York, Academic Press (1971) zusammengestellt. Der Nachteil des Einsatzes von herkömmlichen inneren Prallblechen ist, daß der homogene Fluidisierungszustand verloren geht. Andererseits ist schon vorgeschlagen worden, grobe Feststoffteilchen in mehrstufigen Fließbetten mit Prallblechen zu verwenden, um in den zwei Stufen einen Gegenstromkontakt von Gas und Feststoffen zu erhalten, wie P. Guigon u.a. in "Partide Interchance Between Stages in a Battled Fluidized Bed" A.I.CH.E. Symp. Ser. 70, Seite 63 (1974) beschreiben. Die hohe Austauschrate an Feststoffteilchen in den beiden Stufen eines mit Prallblechen versehenen Fließbettes ist bei homogener Fluidisierung nicht durchführbar. Dies ist aber ein Versuch, einen Gegenstrom von Feststoffen und Gas zu erhalten.

Vielfach-Fließbetten in vertikaler Anordnung sind mit Feststoffteilchen-Transport zwischen den Fließbetten verwendet worden. Nach dem Stand der Technik sind schon verschiedene Systeme mit Prallblechen zur mechanischen Verteilung von Flüssigkeiten verwendet worden, um einen besseren Flüssigkeits-Feststoff-Kontakt zu erhalten. Es ist bekannt, in einer Vielzahl vertikal angeordneter Fließbette Überlauffallrohre vorzusehen, die Feststoffe von einem höherliegenden Fließbett zu einem tieferliegenden Fließbett zu transportieren. Die US-PS 2 639 973 zeigt Überlauffall-

0106944

rohre für Feststoffe zum unteren Teil eines tieferliegenden Fließbettes.
Die US-PSn 2 684 840, 2 698 321, 2 891 846, 2 890 106, 3 910 769 und die
FR-PS 1 058 923 zeigen Überlauffallrohre zum Transport von Feststoffen,
die von der höherliegenden Oberfläche eines höherliegenden Fließbettes
zum mittleren oder unteren Teil eines tieferliegenden Fließbettes führen.
Das US-PS 4 017 585 zeigt den Feststoff-Transport von dem unteren Teil
eines höherliegenden Fließbettes oberhalb der Oberfläche eines tieferliegenden Fließbettes. Die angeführten Patente lassen den Wunsch erkennen, den Gas-Feststoff-Kontakt der Fließbette zu verbessern und die
Reaktionsbedingungen in dem Fließbett zu steuern.

Der Feststoff-Transport für eine Wärmeversorgung zwischen zwei vertikal
angeordneten Fließbetten, wobei Steigrohre von dem oberen Teil eines
tieferliegenden Fließbettes zum unteren Teil eines benachbarten höherliegenden Fließbettes und Fallrohre vom unteren Teil eines höherliegenden Fließbettes zum unteren Teil eines benachbarten tieferliegenden
Fließbettes verwendet werden, ist in der Veröffentlichung von H. Kono
und K. Ninomiya "Heat Supply System for a Fluidized Bed Reactor Featuring an Endothermic Reaction" A.I.CH.E. 20th National Heath Transfer
Conference, Milwaukee, W.s. Aug. 2-5, 1981, beschrieben.

Es ist Aufgabe der Erfindung, ein Verfahren und eine Vorrichtung zur
Durchführung von Reaktionen in Fließbett-Feststoffsystemen zu schaffen,
die selbst bei hohen Oberflächengasgeschwindigkeiten, welche bis zu
dreimal größer sind als die Endgeschwindigkeit der Feststoffteilchen,
eine lineare Druckverteilung entlang der vertikalen Achse des Fließbettes aufweisen, so daß sich dichte, homogene Fließbettzonen mit einem
Leerraum über denselben einstellt. Außerdem soll eine hohe Feststoffzirkulation zwischen den Fließbetten und ein Gegenstrom von Feststoff und
Gas erreicht werden, wobei der Grad der Gasrückmischung gesteuert werden
kann. Ohne Änderung der Kenndaten soll eine Labor- oder Versuchsanlage
in eine industrielle Anlage ausgebaut werden können.

A 5144 - 4 - 0106944

Diese Aufgabe wird nach der Erfindung durch ein Verfahren gelöst, das dadurch gekennzeichnet ist, daß ein Aufwärtsstrom eines Fluidisierungsgases über eine Vielzahl vertikal angeordneter Fließbettzonen mit einer Geschwindigkeit aufrecht erhalten wird, um die Feststoffe in allen Fließbettzonen in fluidisiertem Zustand zu halten und über den fluidisierten Feststoffen in den Fließbettzonen einen Leerraum einzuhalten, daß die Feststoffe zwischen den Fließbettzonen über Fallrohre von dem unteren Teil einer dieser Fließbettzonen zu dem unteren Teil einer tieferliegenden Fließbettzone gemischt werden und daß die Feststoffe auch von unten nach oben zwischen den Fließbettzonen gemischt werden, während die Vorrichtung so aufgebaut ist, daß in einem Kessel ein Aufwärtskanal für das Fluidisierungsgas gebildet ist, daß Mittel zur Einführung und Ableitung des Fluidisierungsgases am Kessel angebracht sind, daß im Kessel eine Vielzahl von vertikal angeordneter Fließbettzonen gebildet sind, die jeweils eine Grundprallplatte haben, welche eine Vielzahl von Öffnungen heben und die unteren Grenzen der Fließbettzonen festlegen und daß mindestens ein Fallrohr vorgesehen ist, das eine obere Öffnung in dem unteren Teil einer Fließbettzone hat und sich mit seiner unteren Öffnung in den unteren Teil einer tieferliegenden Fließbettzone erstreckt.

Vorteilhafte Weiterbildungen des Verfahrens und der Vorrichtung sind den Unteransprüchen entnehmbar.

Mit diesem Verfahren und dieser Vorrichtung bildet jede Fließbettzone eine dichte, homogene und stabile Phase, wobei über jeder dieser Phasen ein Leerraum entsteht, wenn Teilchen verwendet werden, die einen Durchmesser kleiner als $100\mu$ haben. Das Verfahren und die Vorrichtung nach der Erfindung eignen sich mit Vorteil in Vielfach-Fließbetten in chemischen Katalysatorsystemen, um ein hohes Trennvermögen zu erhalten. Auerdem ist die Verweildauer des Gases in einer Fließbettzone wesentlich kleiner als bei herkömmlichen Fließbetten.

A 5144 - 5 -

0106944

Die Erfindung wird anhand von in der Zeichnung dargestellten Ausführungsbeispielen näher erläutert. Es zeigt:

Fig. 1 einen schematischen Längsschnitt durch ein Vielfach-Fließbettsystem mit Fallrohren nach einem ersten Ausführungsbeispiel
der Erfindung,

Fig. 2 eine schematische Perspektivansicht eines Vielfach-Fließbettsystems mit Fallrohren und Steigrohren nach einem anderen
Ausführungsbeispiel und

Fig. 3 ein schematischer Teilschnitt durch ein vertikal und
horizontal vervielfachtes Fließbettsystem nach einem weiteren
Ausführungsbeispiel.

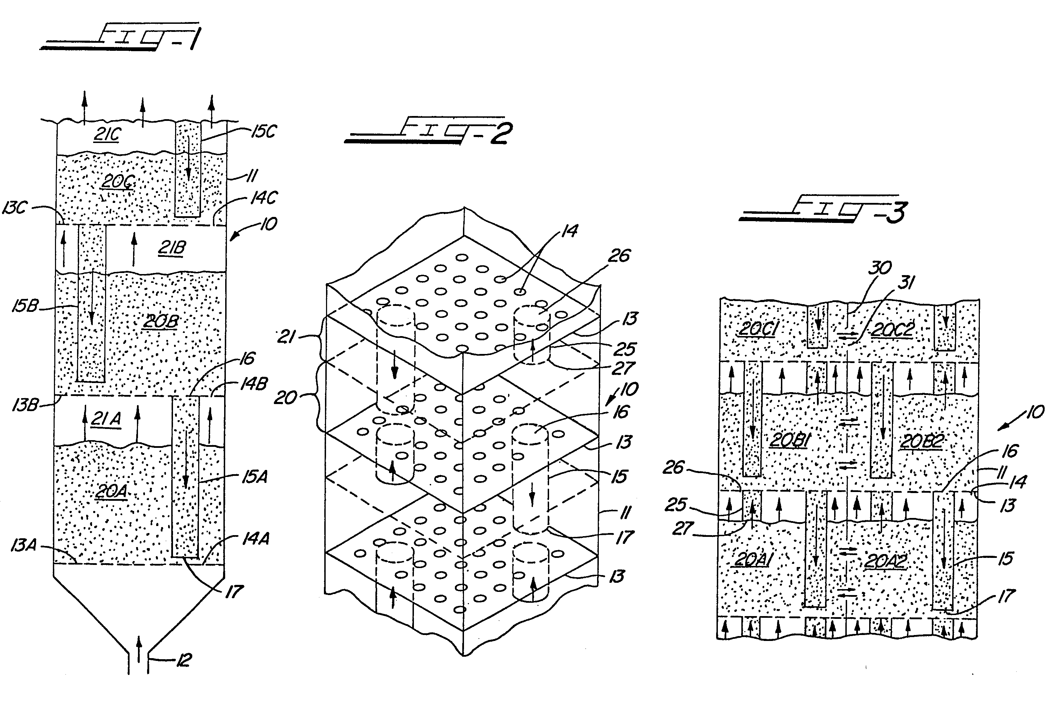

Fig. 1 zeigt den Fließbettreaktor 10, der sich in dem Kessel 11 mit am
unteren Ende angeordneter Eingangsleitung 12 für das Fluidisierungsgas
befindet. Eine ähnliche Ausgangsleitung für das Fluidisierungsgas befindet sich am oberen Ende des Kessels 11, um das durch den Kessel 11
aufwärts strömende Fluidisierungsgas abzuführen. Die Steuerung und das
Unterdrucksetzen des Fluidisierungsgases werden nicht näher beschrieben,
da jedes geeignete Druck- und Regelventil verwendet werden kann, um die
gewünschte Durchflußmenge an Fluidisierungsgas in dem Fließbettreaktor
zu erhalten. In gleicher Weise ist der Kreislauf des Fluidisierungsgases
sowie der Entzug der Feststoffteilchen aus dem Fluidisierungsgas aus dem
Stand der Technik bekannt und wird in der Beschreibung nicht mehr näher
erläutert, da der Grad des Entzuges von Teilchen von der betreffenden
speziellen Teilchenreaktion abhängt. Über nahezu den gesamten Querschnitt des Kessels 11 werden Grundprallplatten, die mit 13A, 13B und
13C bezeichnet sind, in Abständen zueinander angeordnet, um für jede
Fließbettzone 20A, 20B und 20C eine untere Abgrenzung zu erhalten. Jede
Grundprallplatte hat eine Vielzahl von Öffnungen 14A, 14B bzw. 14C, die den Aufwärtsstrom des Fluidisierungsgases zulassen. Die Ausbildung der Öffnung 14 kann in an sich bekannter Weise in jeder gewünschten Form vorgenommen werden. Die Größe der einzelnen Öffnungen und der gesamte Öffnungsquerschnitt der Grundprallplatte können in allen Grundprallplatten gleich oder unterschiedlich sein, um die gewünschten Verbindungen zwischen derVielzahl der Fließbettzonen zu erhalten. Diese Verbindung hängt von der Größe der Feststoffteilchen ab, von dem Vorsehen oder Nichtvorsehen von Fallrohren, von dem Querschnitt der Fallrohre und von der gewünschten Verweilzeit der Feststoffteilchen in jeder Fließbettzone.

Fig. 1 zeigt ein Ausführungsbeispiel der Erfindung, bei dem keine Steigrohre für den Aufwärtstransport von Feststoffteilchen von einer tieferliegenden zu einer höherliegenden Fließbettzone vorgesehen sind. Die Aufwärtsbewegung der Feststoffteilchen von einer Fließbettzone zur nächst höherliegenden Fließbettzone wird dadurch erreicht, daß die Feststoffteilchen von dem Fluidisierungsgasstrom mitgerissen werden, der von der tieferliegenden Fließbettzone zu der nächst höherliegenden Fließbettzone strömt, wie die Pfeile in Fig. 1 zeigen. In dem Ausführungsbeispiel werden die Feststoffteilchen in Fallrohren 15A, 15B und 15C abwärts bewegt, die sich jeweils von dem unteren Teil der unmittelbar angrenzenden, höherliegenden Fließbettzone in den unteren Teil der jeweils benachbarten tieferliegenden Fließbettzonen erstrecken. Ein wichtiger Bestandteil dieser Erfindung ist die große Menge an bewegten Feststoffteilchen zwischen den vertikal angeordneten Fließbettzonen, die durch die Fallrohre, die sich jeweils von dem unteren Teil einer höherliegenden Fließbettzone in den unteren Teil einer tieferliegenden Fließbettzone erstrecken, erreicht wird. Es hat sich als sehr wesentlich herausgestellt, daß die obere Öffnung 16 des Fallrohres 15A und die untere Öffnung 17 des Fallrohres 15A jeweils in dem unteren 5-tel der Höhe der zugeordneten Fließbettzonen 20A bzw. 20B angeordnet sind, damit die Vorteile der Erfindung am besten erreicht werden.

Wie gezeigt ist, befindet sich die obere Öffnung 16 des Fallrohres 15A direkt in der Grundprallplatte 13B der Fließbettzone 20B. Nach einer bevorzugten Ausgestaltung ist die obere Öffnung des Fallrohres in der Grundprallplatte angeordnet, während sich die untere Öffnung des Fallrohres im unteren 10-tel der Fließbettzone befindet. Es hat sich gezeigt, daß durch das in der beschriebenen Stellung befindliche Fallrohr der Betrag des Zirkulationsstromes an Feststoffteilchen zwischen den Fließbettzonen durch die Ausgestaltung des Fallrohres bestimmt wird. In Übereinstimmung mit der Erfindung nimmt bei zunehmendem Durchmesser des Fallrohres das Zirkulationsvolumen an Feststoffteilchen ebenfalls zu. Nach einem anderen Ausführungsbeispiel kann das Fallrohr ein ringförmiger Kanal auf der Innenseite des Kessels um die Fließbettzone sein. Dieser Kanal hat seine obere Öffnung im unteren Bereich einer Fließbettzone und erstreckt sich bis in den unteren Bereich einer tieferliegenden Fließbettzone.

Der in Fig. 1 schematisch dargestellte Prozeß kann mit jedem gewünschten Material an Feststoffteilchen ausgeführt werden. Die Größe der Feststoffteilchen hängt von der Dicke des verwendeten Materials ab. Die Feststoffteilchen müssen aber klein genug sein, damit der aufwärts gerichtete Fluidisierungsstrom genügend Feststoffteilchen mitreißt und zwischen den Fließbettzonen transportiert, während ein Bettraum an diskreten, dichten und homogenen Feststoffteilchen erhalten bleibt. Im allgemeinen haben die Teilchen einen durchschnittlichen Durchmesser kleiner als 100μ und können nach der Erfindung ohne Steigrohre verwendet werden. Entsprechend der Erfindung ist es erwünscht, eine durchschnittliche Verweilzeit zwischen ca. 10 bis 50 Sekunden, vorzugsweise ca. 25 bis 35 Sekunden, der Feststoffteilchen in einer Fließbettzone einstellen zu können.

Die Verweildauer wird bei jedem speziellen Feststoff-Material durch den gesamten Fallrohrquerschnitt, den Gesamtquerschnitt der Öffnungen in der Grundprallplatte, den Fließbettquerschnitt und die Geschwindigkeit des Fluidisierungsgases bestimmt. Für besondere Prozesse kann die durchschnittliche Verweildauer der Feststoffteilchen in jeder Fließbettzone verschieden sein. Dies wird durch die Veränderung des Fallrohrquerschnittes und des Querschnittes der Öffnungen in der Grundprallplatte der verschiedenen Fließbettzonen, sowie durch Veränderung des Hauptmaterials der Feststoffteilchen erreicht. In Fig. 1 ist für jede Fließbettzone ein einziges Fallrohr 15A, 15B und 15C eingesetzt. Es ist einleuchtend, daß jede beliebige Anzahl von Fallrohren verwendet werden kann, um das Volumen, die Geschwindigkeit und die Verteilung des abwärts gerichteten Teilchenstromes, welche für die Durchführung der verschiedenen Prozesse in den Fließbettzonen erforderlich sind, zu erreichen. In der Regel sind zwischen 1 und 6 Fallrohre pro Fließbettzone geeignet. Der große Volumenstrom an Feststoffteilchen zwischen den Fließbettzonen ist ein wesentlicher Punkt, damit eine homogene Fluidisierung bei größerer Oberflächengasgeschwindigkeit erhalten wird. Die Fließbettzonen nach der Erfindung arbeiten ganz anders als herkömmliche, mehrstufige Fließbetten, da sie einen stabilen dichten Fluidisierungszustand bei viel größeren Gasgeschwindigkeiten durch Zunahme und Steuerung der Zirkulationsrate der Feststoffteilchen zwischen den Vielfach-Fließbetteinheiten aufrechterhalten.

Fig. 2 zeigt schematisch eine andere Vorrichtung nach der Erfindung, die ähnlich der in Fig. 1 gezeigten Vorrichtung aufgebaut ist, sie ist aber mit Steigrohren 25 ausgestattet. Die unteren Öffnungen 27 der Steigrohre 25 befinden sich im oberen Teil, vorzugsweise im oberen 10-tel der Höhe der Fließbettzone, oder über der Oberfläche der tieferliegenden Fließbettzone, während die obere Öffnung 26 der Steigrohre 25 im unteren Teil einer höherliegenden Fließbettzone angeordnet sind. Die Steigrohre 25 verbessern den Aufwärtstransport der Feststoffteilchen und sie sind dann erforderlich, wenn in den Fließbettzonen große Teilchen verwendet werden. Obwohl das Steigrohr 25 in Fig. 2 auf gleicher Ebene wie das Fallrohr gezeigt ist, ist dies nicht unbedingt erforderlich. Das Steigrohr und das Fallrohr können in unterschiedlichen Stellungen an der Grund prallplatte befestigt sein. Es können auch so viele Fall- und Steigrohre in eine Fließbettzone eingesetzt werden, wie für den gewünschten Teilchentransport nötig sind.

Fig. 3 zeigt ein weiteres Ausführungsbeispiel der Erfindung, bei dem horizontale Reihen vertikal angeordneter Vielfach-Fließbettzonen vorgesehen sind. Die Fließbettzonen 20A1, 20B1 und 20C1 sind durch die vertikale Trennwand 30 mit den Öffnungen 31 von den Fließbettzonen 20A2, 20B2 und 20C2 der folgenden Reihe getrennt. Die in Fig. 3 gezeigte Vorrichtung erlaubt den vertikalen und horizontalen Transport von Feststoffteilchen zwischen diskreten homogenen Flußbettzonen, entsprechend der Erfindung. Der horizontale Transport von Feststoffteilchen geschieht im wesentlichen auf gleicher Höhe horizontal benachbarter Fließbettzonen. Die Fall- und Steigrohre, die in Fig. 3 gezeigt sind, entsprechen den in Fig. 2 gezeigten und beschriebenen Fall- und Steigrohren. Horizontale Reihen von vertikal angeordneter Vielfach-Fließbettzonen nach der Erfindung schaffen eine Möglichkeit, kommerzielle Anlagen mit getrennten Einheiten aufzubauen, die ähnliche Kenndaten wie Labor- oder Versuchsanlagen haben.

Werden kleinere Feststoffteilchen (100µ und kleiner) in dem Fließbettsystem nach der Erfindung verwendet, kann der Aufwärtstransport der Feststoffteilchen eine Kombination aus Mitreißen im Gasstrom und dem Strom in den Steigrohren sein, wie die Pfeile in Fig. 3 zeigen. Der Aufwärtstransport der Feststoffteilchen kann in einem Steigrohr erfolgen, welches sich durch eine nach oben angrenzende Fließbettzone bzw. Fließbettzonen hindurch in eine höherliegende Fließbettzone erstreckt. Genauso kann der abwärts gerichtete Transport der Feststoffteilchen durch ein Fallrohr erfolgen, das sich durch eine nach unten anschließende Fließbettzone bzw. Fließbettzonen hindurch in eine nicht angrenzende, tieferliegende Fließbettzone erstreckt.

Die Fließbettzonen nach der Erfindung ermöglichen eine homogene Fluidisierung mit dichten Fließbetten. Aufgrund der mit der Erfindung erreichten homogenen, dichten Fließbettzonen wird die Ausbaumöglichkeit von Labor- oder Versuchsanlagen zu Werksanlagen erreicht, wobei die Fließbettzonen in gleicher Weise arbeiten. Die Fließbettzonen nach der Erfindung sehen ein gesteuertes Mischungsverhältnis von Gas und Feststoffen mit engem Feststoff-Gas-Kontakt vor. Die Vielzahl von vertikal angeordneten Fließbettzonen mit einem großen Volumen an Feststoffteilchen-Transport zwischen den Fließbettzonen führt zu einem Gas-Feststoff-Gegenstrom-Kontakt. Die Fließbettzonen nach der Erfindung beinhalten alle vorgenannten Charakteristiken eines Vielfach-Fließbettes, wie sie bisher mit den herkömmlichen Vielfach-Fließbettsystemen, insbesondere bei kleinen Feststoffteilchen mit einem durchschnittlichen Durchmesser kleiner als 100μ, noch nicht erreicht worden sind.

Geeignete Materialien für den Aufbau der Vorrichtung nach der Erfindung brauchen nicht angegeben zu werden, da ein Fachmann auf diesem Gebiet die Materialien ohne weiteres aussucht, so daß eine weitere Erörterung unterbleiben kann.

Dies liegt auch in der großen Vielfalt der geeigneten Materialien und in der Vielzahl der unterschiedlichen thermischen oder chemischen Reaktionen begründet, die in der Vorrichtung durchgeführt werden.

Die folgenden Beispiele werden als weitere Ausführungsformen fortgesetzt. Die Verwendung spezieller Materialien oder Bedingungen darf nicht als Beschränkung der Erfindung betrachtet werden.


Beispiel I

Um eine Vielzahl von vertikal angeordneten Fließbettzonen entsprechend einem Ausführungsbeispiel der Erfindung zu zeigen, wurde ein Kessel mit einem Durchmesser von 5 cm und einer Höhe von 350 cm mit Grundprallplatte und Fallrohren nach Fig. 1 gebaut. Der Kessel hat 12 Grundprallplatten, die jeweils im gleichen Abstand von 6 cm angeordnet sind. Jede Grundprallplatte hat 10 runde Steigrohröffnungen mit einem Durchmesser von 0,5 cm. Die unterste Grundprallplatte wurde mit einem Gasverteiler für Fluidisierungsgas verbunden. Ein Fallrohr, wie in Fig. 1 gezeigt, erstreckt sich von einer höherliegenden Grundprallplatte 5 cm nach unten und hat einen Durchmesser von 2,5 cm. An jeder Grundprallplatte, mit Ausnahme der untersten, ist so ein Fallrohr angebracht. Die Feststoffteilchen in einem Katalysator für katalytisches Wirbelschichtkracken haben einen Teilchendurchmesser von 65$\mu$ und werden bis zu einer Höhe von 30 cm von der untersten Grundprallplatte eingefüllt. Die kleinste Fluidisierungsgeschwindigkeit war 0,25 cm/Sekunde bei 20° C und die Oberflächengasgeschwindigkeit betrug 30 cm/Sekunde bei 20° C. Unter diesen Arbeitsbedingungen wurde entlang der vertikalen Achse des Fließsystems die Druckverteilung ermittelt und als Druckdifferenz zwischen dem gemessenen Druck und dem atmosphärischen Druck in mm Wassersäule angegeben. Die Tabelle I zeigt die Ergebnisse:

Tabelle I

| Abstand des Meßpunktes vom Gasverteiler (cm) | Druck P (mm $H_2O$) |
|---|---|
| 29 | 218 |
| 59 | 178 |
| 89 | 138 |
| 119 | 98 |
| 149 | 58 |
|  | 18 |
| 209 | 0 |

Die Daten in der Tabelle zeigen, daß unter den Fluidisierungsbedingungen in der Vorrichtung nach der Erfindung gut stabilisierte Fließbettbedingungen erreicht werden können. In jeder Fließbettzone wurde ein stabilisiertes Fließbett gebildet.

Im Vergleich dazu war ein konventionelles Fließbett, das gleich große Teilchen verwendete, aber keine Grundprallplatte und Fallrohre besaß, bei gleichen Bedingungen wie bei der Vorrichtung nach der Erfindung aufgrund des intensiven Perlens weit unterlegen, da sich keine stabilisierten Fließbette bildeten.

## Beispiel II

Um die Steuerung der Gasrückmischung zu zeigen, die sich bei der Vorrichtung und dem Prozeß nach der Erfindung einstellt, wurde ein Kessel mit einem Durchmesser von 15 cm und einer Höhe von 240 cm mit 6 Grundprallplatten gebaut. Die Grndprallplatten werden in Abständen von 30 cm angeordnet. Jede Grundprallplatte hat 15 Steigrohröffnungen mit einem Durchmesser von 1,3 cm. Ein einzelnes Fallrohr erstreckt sich von jeder höherliegenden Grundprallplatte über 29 cm nach unten. Seine untere Öffnung liegt 1 cm über der tieferliegenden Grundprallplatte und hat einen Durchmesser von 7 cm. Teilchen für ein katalytisches Wirbelschichtkracken mit einer durchschnittlichen Größe von 65µ im Durchmesser wurden bis zu einer Höhe von 90 cm vor der Fluidisierung eingefüllt. Die kleinste Fluidisierungsgeschwindigkeit betrug 0,25 m/Sekunde bei 20° C und die Oberflächengasgeschwindigkeit war 20 cm/Sekunde bei 20° C. In die vierte Fließbettzone über dem Gasverteiler an der untersten Grundprallplatte wurde Tracer-$CO_2$-Gas eingespritzt und die Konzentrationen des $CO_2$-Gases in vertikaler Richtung bei gleichbleibendem Zustand gemessen. Aufgrund dieser Messungen wurden die Gasrückmischungskoeffizienten nach dem Verfahren errechnet, das F.J. Zuiderweg in dem Abschnitt "mixing" des Buches von J.F. Davidson u.a. "Fluidization", Seite 347, New York, Academic Press, 1971, beschrieben hat.

Ein zweites Fließbett wurde ohne Grundprallplatte und Fallrohre hergestellt. In diesem Fließbett wurde an der gleichen Stelle das Tracer-Gas eingespritzt und die Gas-Rückmischungskoeffizienten errechnet. Für das Fließbettsystem nach der Erfindung wurde ein Gas-Rückmischungskoeffizient von 60 cm³/Sekunde errechnet, während der Gas-Rückmischungskoeffizient in einem einzigen herkömmlichen Fließbett bei 500 cm³/Sekunde liegt. Daraus wird deutlich, daß der Gas-Rückmischungskoeffizient durch die Vorrichtung und den Prozeß nach der Erfindung auf einem wesentlich niedrigerem Pegel gehalten werden kann.


Beispiel III

In einem Kessel ist eine Vielzahl vertikal angeordneter Fließbettzonen untergebracht. Der Kesselhat einen Durchmesser von 10 cm und eine Höhe von 300cm. In Abständen von 30 cm werden 6 Grundprallplatten in den Kessel eingebaut. Jede Grundprallplatte hat 12 Steigrohröffnungen mit einem Durchmesser von 1,2 cm. Jede Fließbettzone, außer der untersten, hat ein Fallrohr, das von der Grundprallplatte ausgeht und sich 37 cm nach unten erstreckt. Das Fallrohr hat einen Durchmesser von 5 cm.

Das Fließbettsystem wurde für die katalytische Wasserstoffentziehung bei der Gewinnung von Propen aus Propan verwendet. Als Katalysator wurden 7 Gewichtsprozente $Cr_2O_3$ und 93 Gewichtsprozente $Al_2O_3$ in gammakristalliner Form verwendet. Die durchschnittliche Teilchengröße betrug 70μ. Die Einheit wurde vor der Fluidisierung mit den festen Katalysatorteilchen bis zu 90 cm Betthöhe gefüllt. Die kleinste Fluidisierungsgeschwindigkeit betrug 0,3 m/Sekunde bei 620° C und Oberflächengasgeschwindigkeit 30 cm/Sekunde bei 620° C. Vergleichsweise wurde mit gleich großen Teilchen und unter gleichen Reaktionsbedingungen eine Umwandlung von Propan in Propen bei 620° C in einem herkömmlichen Fließbettsystem durchgeführt.

Die Tabelle III zeigt die Daten, die aus dem Vergleich zwischen dem herkömmlichen Fließbettsystem und einem Fließbettsystem mit einer Vielzahl vertikal angeordneter Fließbettzonen nach der Erfindung erhalten wurden.

Tabelle III

| Vielzahl vertikal angeordneter Fließbettzonen | | herkömmliches einfaches Fließbett |
|---|---|---|
| Umwandlung (%) | 52,9 | 53,1 |
| Trennvermögen (%) | 75,4 | 60,2 |
| Gewinn in einem Durchlauf (%) | 40,0 | 30,0 |

Die Tabelle III zeigt, daß das Trennvermögen bei der Vorrichtung und dem Prozeß nach der Erfindung deutlich zugenommen hat. Die Zunahme des Trennvermögens ist wahrscheinlich das Ergebnis der reduzierten Gas-Rückmischung, die aus der unvorteilhaften Sekundärreaktion beim Kracken von Propen entsteht. Es muß auch darauf hingewiesen werden, daß der Gewinn bei einem Durchlauf um mehr als 25 % zugenommen hat.

Die Erfindung wurde vorstehend anhand von bestimmten, bevorzugten Ausführungsbeispielen in vielen Details zum Zwecke der Erläuterung beschrieben. Für den Fachmann auf diesem Gebiet ist es selbstverständlich, daß die Erfindung auch in anderen zusätzlichen Ausführungsarten anwendbar ist und daß bestimmte Einzelheiten wesentlich variiert werden können, ohne von dem Grundgedanken der Erfindung abzuweichen.

A 5144
v/p

Institute of
Gas Technology
3424 South State Street

Chicago, Illin. 60616
U.S.A.

- 1 -


Patentansprüche


1.  Verfahren zur Durchführung von Reaktionen in Fließbett-Feststoffsystemen,
    dadurch gekennzeichnet,
    daß ein Aufwärtsstrom eines Fluidisierungsgases über eine Vielzahl
    vertikal angeordneter Fließbettzonen mit einer Geschwindigkeit
    aufrechterhalten wird, um die Feststoffe in allen Fließbettzonen
    in fluidisiertem Zustand zu halten und über den fluidisierten
    Feststoffen in den Fließbettzonen einen Leerraum einzuhalten,
    daß die Feststoffe zwischen den Fließbettzonen über Fallrohre von
    dem unteren Teil einer dieser Fließbettzonen zu dem unteren Teil
    einer tieferliegenden Fließbettzone gemischt werden und
    daß die Feststoffe auch von unten nach oben zwischen den Fließbettzonen gemischt werden.


2.  Verfahren nach Anspruch 1,
    dadurch gekennzeichnet,
    daß die Mischung der Feststoffe von oben nach unten über Fallrohre
    durchgeführt wird.

A 5144          - 2 -          0106944

3.     Verfahren nach Anspruch 1,
dadurch gekennzeichnet,
daß die Mischung der Feststoffe von unten nach oben zwischen den Fließbettzonen durch Mitreißen beim Durchgang des von unten nach oben strömenden Fluidisierungsgases vorgenommen wird.

4.     Verfahren nach Anspruch 3,
dadurch gekennzeichnet,
daß das Fluidisierungsgas durch mindestens ein Steigrohr geleitet wird, das eine untere Öffnung in dem unteren Teil oder über der Oberfläche einer der Fließbettzonen hat und sich mit seiner oberen Öffnung in den unteren Teil einer höherliegenden Fließbettzone erstreckt.

5.     Verfahren nach einem der Ansprüche 1 bis 4,
dadurch gekennzeichnet,
daß in horizontalen Reihen einer Vielzahl vertikal angeordneter Fließbettzonen durch fluidisierte Feststoffe zwischen benachbarten horizontalen Fließbettzonen im wesentlichen gleiche Fließbetthöhen eingehalten werden.

6.     Verfahren nach einem der Ansprüche 1 bis 5,
dadurch gekennzeichnet,
daß fluidisierte Feststoffe mit einer Teilchengröße mit einem durchschnittlichen Durchmesser kleiner als 100µ verwendet werden.

7.     Vorrichtung zur Durchführung des Verfahrens nach einem der Ansprüche 1 bis 6,
dadurch gekennzeichnet,
daß in einem Kessel (11) ein Aufwärtskanal für das Fluidisierungsgas gebildet ist,
daß Mittel zur Einführung und Ableitung des Fluidisierungsgases am Kessel (11) angebracht sind, daß im Kessel (11) eine Vielzahl von vertikal angeordneter Fließbettzonen (20A,20B,20C) gebildet sind, die jeweils eine Grundprallplatte (13A,13B,13C) haben, welche eine Vielzahl von Öffnungen (14A,14B,14C) heben und die unteren Grenzen der Fließbettzonen (20A,20B,20C) festlegen und

daß mindestens ein Fallrohr (z. B. 15A) vorgesehen ist, das eine obere Öffnung (16) in dem unteren Teil einer Fließbettzone (z. B. 20B) hat und sich mit seiner unteren Öffnung (17) in den unteren Teil einer tieferliegenden Fließbettzone (z. B. 20A) erstreckt.

8. Vorrichtung nach Anspruch 7,
dadurch gekennzeichnet,
daß die obere Öffnung (16) und die untere Öffnung (17) des Fallrohres (15A) jeweils in dem untersten 5-tel, vorzugsweise 10-tel, der Höhe der zugeordneten Fließbettzonen (20B und 20A) angeordnet sind.

9. Vorrichtung nach Anspruch 7,
dadurch gekennzeichnet,
daß die obere Öffnung (16) des Fallrohres (15A) direkt in der Grundprallplatte (13B) und die untere Öffnung (17) des Fallrohres (15A) in dem untersten 5-tel, vorzugsweise 10-tel, der Höhe der zugeordneten Fließbettzonen (20B und 20A) angeordnet sind.

10. Vorrichtung nach einem der Ansprüche 7 bis 9,
dadurch gekennzeichnet,
daß jede Fließbettzone (20A,20B,20C) mehr als ein Fallrohr aufweist.

11. Vorrichtung nach einem der Ansprüche 7 bis 10,
dadurch gekennzeichnet,
daß das Fallrohr als Ringkanal am Umfang der Fließbettzone ausgebildet ist.

12. Vorrichtung nach einem der Ansprüche 7 bis 11,
    dadurch gekennzeichnet,
    daß mindestens ein Steigrohr (25) vorgesehen ist, das mit seiner unteren Öffnung (27) im unteren Teil oder über der Oberfläche einer Fließbettzone angeordnet ist und sich mit seiner oberen Öffnung (26) in den unteren Teil einer höherliegenden Fließbettzone erstreckt.

13. Vorrichtung nach Anspruch 12,
    dadurch gekennzeichnet,
    daß zumindest die untere Öffnung (27) des einzigen Steigrohres (25) in dem unteren 5-tel, vorzugsweise 10-tel, der Höhe der zugeordneten Fließbettzone angeordnet ist.

14. Vorrichtung nach Anspruch 12,
    dadurch gekennzeichnet,
    daß zumindest die untere Öffnung (27) des einzigen Steigrohres (25) über der Oberfläche der zugeordneten Fließbettzone angeordnet ist.

15. Vorrichtung nach Anspruch 12,
    dadurch gekennzeichnet,
    daß zumindest die obere Öffnung (26) des einzigen Steigrohres direkt in der Grundprallplatte angeordnet ist.

16. Vorrichtung nach einem der Ansprüche 7 bis 15,
    dadurch gekennzeichnet,
    daß sich die Fallrohre über vertikal benachbarte Fließbettzonen erstrecken.

17. Vorrichtung nach einem der Ansprüche 7 bis 15,
    dadurch gekennzeichnet,

daß sich die Fallrohre durch eine vertikal benachbarte Fließbettzone hindurch bis zu einer tieferliegenden, nicht benachbarten
Fließbettzone erstrecken.

18. Vorrichtung nach einem der Ansprüche 7 bis 15,
dadurch gekennzeichnet,
daß sich die Fallrohre und Steigrohre durch eine vertikal benachbarte Fließbettzone hindurch zu einer tieferliegenden und einer
höherliegenden und nicht benachbarten Fließbettzone erstrecken.

19. Vorrichtung nach einem der Ansprüche 7 bis 18,
dadurch gekennzeichnet,
daß horizontale Reihen mit einer Vielzahl vertikal angeordneter
Fließbettzonen vorgesehen sind, die über vertikale Trennwände (30)
voneinander getrennt sind.

20. Vorrichtung nach Anspruch 19,
dadurch gekennzeichnet,
daß die vertikalen Trennwände (30) Öffnungen (31) aufweisen, die
eine Verbindung zwischen horizontal benachbarten Fließbettzonen
(z. B. 20B1, 20B2) herstellen.

FIG-1

FIG-2

FIG-3